(19) Europäisches Patentamt
European Patent Office
Office européen des brevets

(11) **EP 4 365 383 A2**

(12) **EUROPEAN PATENT APPLICATION**

(43) Date of publication:
**08.05.2024 Bulletin 2024/19**

(51) International Patent Classification (IPC):
***E03D 11/00*** *(2006.01)*

(21) Application number: **24165213.0**

(22) Date of filing: **29.01.2021**

(52) Cooperative Patent Classification (CPC):
**A61B 5/208; A61B 5/4875; A61B 5/6891;
E03D 11/00; G01G 19/52;** A61B 10/0038

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2020 DK PA202070063**

(62) Document number(s) of the earlier application(s) in
accordance with Art. 76 EPC:
**21704705.9 / 4 096 525**

(71) Applicant: **Measurelet ApS
2970 Hørsholm (DK)**

(72) Inventors:
• **BAGGER, Marie Lommer
2830 Virum (DK)**
• **BO SØNDERGAARD SVENDSEN, Morten
2800 Kongens Lyngby (DK)**

(74) Representative: **AWA Denmark A/S
Strandgade 56
1401 Copenhagen K (DK)**

Remarks:
This application was filed on 21.03.2024 as a
divisional application to the application mentioned
under INID code 62.

(54) **A FLUID BALANCE MONITORING SYSTEM AND A METHOD FOR DETERMINING AND MONITORING THE FLUID BALANCE OF A SUBJECT**

(57) A fluid balance monitoring system (200) adapted for determining and monitoring the fluid balance of a subject (100), the fluid balance monitoring system (200) comprising at least one input unit (202) configured to receive measurement data comprising information relevant for determining and monitoring the fluid balance of a mammalian subject (100), and transmit the received measurement data to at least one data processing unit (201) configured to receive the measurement data, process the received measurement data to achieve output data indicative of the fluid balance of the subject (100), and transmit the output data to at least one display unit (203) configured to receive the output data and display the output data. The measurement data comprises information regarding a quantity, a type and a time of occurrence for at least one event relevant for determining and monitoring the fluid balance of the subject (100). The data processing unit (201) is configured to process the received measurement data to achieve output data which is indicative of a type and a time of occurrence of the at least one event.

FIG. 2

**EP 4 365 383 A2**

**Description**

**Technical Field**

The present invention relates to a fluid balance monitoring system configured for determining and monitoring the fluid balance of a mammalian subject, the fluid balance monitoring system comprising at least one data processing unit, at least one input unit and at least one display unit. The invention further relates to a method for determining and monitoring the fluid balance of a mammalian subject.

As used herein, the term "excretion", when used as a noun, is intended to encompass in principle any waste product, solid or liquid, that is eliminated from a human body, but particularly urine and faeces. Likewise, as used herein the term "excretion" and conjugations thereof, when used as a verb, is intended to encompass the action of expelling in principle any waste product, solid or liquid, that is to be eliminated from a human body, such as, e.g., urine, feces, vomit, saliva or mucus.

As used herein the terms "subject" and "mammalian subject" are intended to encompass in principle any subject for which it is desired to monitor the fluid balance, but particularly such subjects being relevant for the health sector, i.e. mammals such as particularly human beings.

**Background art**

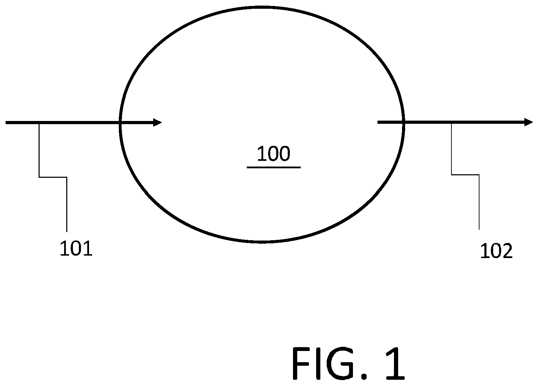

The fluid balance of a subject, such as a mammalian or human subject, affects homeostatic processes such as acid-base balance, ion balance, liquid transport and similar within the subject. The fluid balance of a subject 100 is illustrated schematically on Fig. 1. The fluid balance of a subject 100 expresses the change of water in the subject 100, that is the relation between the inflow 101 or volume of liquid flowing into the subject 100 and the outflow 102 or volume of liquid flowing out of the subject 100. The fluid balance of a subject 100 to a given time can be expressed as

$$\text{Fluid balance} = \text{Inflow} - \text{Outflow}$$

The fluid balance of a subject, such as a human subject, is a parameter of great importance, which within the health sector is frequently used as one parameter amongst others when diagnosing diseases and monitoring progress of diseases.

Liquid can flow into the subject via intake of food or drink or by means of access through bone, vascular system, intestines and even via surfaces if there is a difference in the water vapor pressure, or osmotic pressure, between internally and externally with respect to the subject.

Liquid can flow out of the subject through evaporation (e.g. sweating) from surface areas such as lung tissue or skin tissue, or via expelling excretions such as urine and feces. Further, liquid can flow out of the subject orally via sputum, ulcers, drains, mucosal, and gastric evacuations.

A simple and often used way of measuring the fluid balance of a subject is by monitoring the mass of the subject. Measuring the mass of a subject can be done by weighing the subject. However, the mass will only signify the present status of the subject and thus only provides a snapshot of the fluid balance of the subject. An increase in mass can occur due to increased inflow with a constant outflow, or similarly, a constant inflow and decreased outflow. Likewise, a decrease in mass can occur due to decreased inflow with a constant outflow, or similarly, a constant inflow and an increased outflow.

Furthermore, the type of inflow and outflow is important, as the water content of different excretions, e.g., feces and urine, differ. Therefore, a determination of type of outflow is important to properly assess the amount of water flowing out of a subject. Likewise, for inflow, the water content of a similar mass of different substances consumed, e.g., water and bread, or otherwise flowing into a subject differ. Therefore, a determination of the type of inflow is also important.

Thus, it is important and consequently desired to measure the inflow and outflow separately and to enable determining the type and time of occurrence of each inflow and outflow to understand the dynamics of the fluid balance of the subject.

Also, it is desired to summarize the fluid balance or change in mass of liquid in a subject over a period of time, t, where the change in mass of liquid equals the sum of liquid inflows subtracted the sum of liquid outflows in that period of time and can be expressed as

$$\sum(\text{Fluid balance}, t) = \sum(\text{Inflow}, t) - \sum(\text{Outflow}, t),$$

where the notation $\Sigma(x, t)$ denotes the sum of the parameter x over the time t. The fluid balance can be expressed as an absolute value or as a percentage of subject mass, e.g. a negative percentage if outflow is larger than inflow, 0 % if inflow and outflow are equal or a positive percentage if inflow is larger than outflow.

To fully understand the dynamics of the fluid balance of a subject, all inflow and outflow must be determined. Ideally, this would be done autonomously. However, the technological environments do not always allow this. This makes it necessary to perform registrations of time and type for each time an inflow or an outflow occurs. Typically, this is done using paper and pen, which is however time consuming and unhygienic and very prone to registration errors.

It is thus desired to provide a fluid balance monitoring system and a method for determining and monitoring the fluid balance of a subject alleviating at least some of the above-mentioned and other drawbacks related to the prior art.

Particularly, it is desired to provide a fluid balance monitoring system and a method for determining and monitoring the fluid balance of a subject enabling determining all inflows and outflows of fluids to and from a subject, enabling measuring the inflows and outflows of fluids to and from a subject separately, enabling determining the type and time of occurrence of each inflow and outflow and enabling summarizing the fluid balance or change in mass of liquid in a subject over a period of time such as to provide a precise and comprehensive fluid balance monitoring and an improved or even full understanding of the dynamics of the fluid balance of a subject.

**Summary of Invention**

It is therefore the object of the invention to provide a fluid balance monitoring system and a method for determining and monitoring the fluid balance of a mammalian subject of the type mentioned in the introduction with which becomes possible to determine all inflows and outflows of fluids to and from a subject, measure the inflows and outflows of fluids to and from a subject separately, determine the type and time of occurrence of each inflow and outflow and summarize the fluid balance or change in mass of liquid in a subject over a period of time, such as to provide a precise and comprehensive fluid balance monitoring and an improved or even full understanding of the dynamics of the fluid balance of a subject.

A further object of the invention is to provide such a fluid balance monitoring system and such a method for determining and monitoring the fluid balance of a mammalian subject which further simplifies both the practical data registration process and the work process of health professionals when monitoring a mammalian subject.

A further object of the invention is to provide such a fluid balance monitoring system which saves time and costs, especially within the professional health care systems, such as hospitals, nursing homes and homecare.

The invention is defined by the subject matter of the independent claims. Particular embodiments of the invention are set out in the dependent claims.

The above and other objects are in a first aspect of the invention achieved by means of a fluid balance monitoring system for determining and monitoring the fluid balance of a mammalian subject, the fluid balance monitoring system comprising at least one data processing unit, at least one input unit and at least one display unit. The at least one input unit is configured to receive, from any one or more of a user interface and at least one sensor device, measurement data comprising information relevant for determining and monitoring the fluid balance of a mammalian subject, and transmit the received measurement to the at least one data processing unit. The at least one data processing unit comprises a data processing device, and the at least one data processing unit is configured to receive the measurement data from the at least one input unit, process the received measurement data to achieve output data indicative of the fluid balance of the mammalian subject, and transmit the output data indicative of the fluid balance of the mammalian subject to the at least one display unit. The at least one display unit comprises a data processing device and the at least one display unit is configured to receive the output data indicative of the fluid balance of the mammalian subject, and display the output data indicative of the fluid balance of a subject. The measurement data further comprises information regarding a quantity, a type and a time of occurrence for at least one event relevant for determining and monitoring the fluid balance of the mammalian subject, where the at least one event is any one relevant event leading to an outflow of fluid from the mammalian subject or an inflow of fluid to the mammalian subject, and the at least one data processing unit furthermore is configured to process the received measurement data to achieve output data which is further indicative of a type and a time of occurrence of the at least one event.

Thereby, and especially by providing that the measurement data further comprises information regarding a quantity, a type and a time of occurrence for at least one event relevant for determining and monitoring the fluid balance of a subject, where the at least one event is any one relevant event leading to an outflow of fluid from the subject or an inflow of fluid to the subject, and that the at least one data processing unit furthermore is configured to process the received measurement data to achieve output data which is further indicative of a type and a time of occurrence of the at least one event, a fluid balance monitoring system is provided with which it becomes possible to determine all inflows and outflows of fluids to and from a subject, measure the inflows and outflows of fluids to and from a subject separately, determine the type and time of occurrence of each inflow and outflow and summarize the fluid balance or change in mass of liquid in a subject over a period of time.

Such a system thus enables providing a precise and comprehensive fluid balance monitoring as well as an improved or even full understanding of the dynamics of the fluid balance of a subject. This in turn provides the further advantage of helping professionals to further understand the dynamics of the fluid balance of the subject, to make quicker and more precise diagnoses, and to more closely and precisely monitor subjects with the aim of a faster and better recovery as well as to prevent hospitalizations and other complications that may follow from an unbalanced liquid balance

Furthermore, a fluid balance monitoring system which saves time and costs, especially within professional health care systems, such as at hospitals, is provided herewith. Saving such costs and especially time may also aid in providing the further advantage of helping professionals to make quicker and more precise diagnoses, and to more closely and precisely monitor subjects with the aim of a faster and better recovery.

Also, by providing that the data collection takes place on one unit, the data processing in another unit and the display of data on yet another unit, the fluid balance monitoring system may be centrally managed by the data processing unit. More importantly, with such a fluid balance monitoring system the data registration process and the display and monitoring process, respectively, may take place on separate units. Thereby, the subject or a caretaker may manage the data collection and registration on one unit, and the subject's fluid balance may be monitored on a different unit. Thus, both the practical data registration process and the work process of health professionals when monitoring a subject is simplified considerably.

In an embodiment, the at least one data processing unit is in data transferring connection with the at least one input unit and the at least one display unit in such a way that all data communication between the at least one input unit and the at least one display unit is lead through the at least one data processing unit.

Thereby, it is provided that the fluid balance monitoring system is centrally managed by the data processing unit. This in turn provides for a simplified fluid balance monitoring system with which the data registration process and the display and monitoring process, respectively, may take place on separate units. Thereby, the subject or a caretaker may manage the data collection and registration on one unit, and the subject's fluid balance may be monitored on a different, separate unit. This provides for that the different users of the system are only presented with information relevant for them. Thus, both the practical data registration process and the work process of health professionals when monitoring a subject is simplified even further.

In an embodiment, the measurement data comprises data related to at least one event relevant for determining and monitoring the fluid balance of the mammalian subject, where the at least one event comprises any one or more of: excretion, defecation, urination, intake of food or drink, exercise, perspiration, respiration, sputum secretion, mucosal secretion, output from drain and gastric evacuation.

Taking into account all or most relevant such events in the fluid balance monitoring of a subject provides a further improved fluid balance monitoring in terms of precision and comprehensiveness. This in turn further improves the precision in both diagnosing subjects and in monitoring subjects with the aim of a faster and better recovery.

In an embodiment, at least one of the measurement data and the output data is saved in a cloud-based storage or a storage provided in the data processing unit.

Thereby, a fluid balance monitoring system is provided with which the output data and measurement data may be kept stored centrally such as to enable access to the data by any authorized display unit at any time. This improves the flexibility of the system.

In a further embodiment, at least one of the measurement data and the output data is associated with or encrypted by an individual identification key. Alternatively, or additionally, at least one of the measurement data and the output data may be saved in a cloud-based storage or a storage provided in the data processing unit together with or encrypted by an individual identification key.

Thereby, it is provided that the measurement data and the output data may be associated with a particular subject in a particularly simple manner. Furthermore, it is also ensured that the measurement data and output data is always associated with the correct subject. Thereby, errors which may occur in case of erroneously associating data with a wrong subject may be minimized or altogether avoided.

In an embodiment the individual identification key is an anonymized individual identification key.

Thereby, the identification of the subject may be kept anonymous, at least until display on the display unit, such as to conform with relevant regulations, such as the GDPR-regulations in force within the European Union.

Furthermore, any of the three above-mentioned embodiments related to central data storage, and especially the provision of an individual identification key, provides for a system in which unauthorized personnel may be denied access to the data, for instance based on a separate key authorizing access to the data processing unit or based on the identification key or suitable properties associated with such a key, e.g., an associated key for decryption. Such systems are provided with an improved data security.

In an embodiment, the output data comprises at least data indicative of an inflow of liquid to the mammalian subject, an outflow of liquid from the mammalian subject and a change in the fluid balance of the mammalian subject.

Thereby, the three most important parameters related to the liquid balance of a subject may be displayed in the display unit. Thereby, a precise and easy to understand monitoring of the fluid balance is obtained, which in turn makes it possible to understand the dynamics of the fluid balance of the subject.

In an embodiment, the display unit is configured to display the output data in a manner showing the data indicative of an inflow of liquid to the mammalian subject, an outflow of liquid from the mammalian subject and a change in the fluid balance of the mammalian subject separately from one another.

Thereby, a fluid balance monitoring system is provided with which it becomes possible to not only determine and measure all inflows and outflows of fluids to and from a subject and measure the inflows and outflows of fluids to and from a subject separately, but also to visualise and display the said inflows and outflows separately together with the fluid balance of the subject, such as to provide a precise and comprehensive fluid balance monitoring being easy to interpret for the viewer, such as a health professional.

In an embodiment, the data processing unit is configured to generate output data and send the output data to the at least one display unit in real time or with predetermined time intervals. Alternatively, or additionally, the display unit is configured to display the output data in real time or with predetermined time intervals.

Thereby, real time monitoring of the fluid balance of a subject is enabled in a particularly simple, quick and easy to use manner.

The above and other objects are in a second aspect of the invention achieved by means of a method for determining and monitoring the fluid balance of a mammalian subject, the method comprising the steps of:

a) providing a fluid balance monitoring system according to any one of the above claims and comprising at least one data processing unit with at least one data processing device, at least one input unit and at least one display unit with at least one data processing device,

b) receiving, with the at least one input unit and from any one or more of a user interface and at least one sensor device, measurement data comprising information relevant for determining and monitoring the fluid balance of a mammalian subject, and

c) transmitting, with the at least one input unit, the received the measurement data to the at least one data processing unit,

d) receiving, with the at least one data processing unit, the measurement data from the at least one input unit,

e) processing, with the at least one data processing unit, the received measurement data to achieve output data indicative of the fluid balance of the mammalian subject,

f) transmitting, with the at least one data processing unit, the output data indicative of the fluid balance of the mammalian subject to the at least one display unit,

g) receiving, with the at least one display unit, the output data indicative of the fluid balance of the mammalian subject, and

h) displaying, with the at least one display unit, the output data indicative of the fluid balance of the mammalian subject,

the measurement data further comprising information regarding a quantity, a type and a time of occurrence for at least one event relevant for determining and monitoring the fluid balance of the mammalian subject, where the at least one event is any one relevant event leading to an outflow of fluid from the mammalian subject or an inflow of fluid to the mammalian subject, and the method comprising the further step of processing, with the at least one data processing unit, the received measurement data to achieve output data which is further indicative of a type and a time of occurrence of the at least one event.

In some embodiments, the method comprises one or more of the further steps of:

saving at least one of the measurement data and the output data in a cloud-based storage or a storage provided in the data processing unit,

associating, providing or encrypting at least one of the measurement data and the output data with an individual identification key,

generating, with the data processing unit, output data in real time and sending, with the data processing unit, the output data to the at least one display unit in real time or with predetermined time intervals, and

displaying, with the display unit, the output data in real time or with predetermined time intervals.

It is noted that the invention relates to all possible combinations of features recited in the claims.

**Brief Description of Drawings**

In the following description embodiments of the invention will be described with reference to the schematic drawings, in which

Fig. 1 is a schematic illustration of the liquid inflow and outflow of a subject, particularly a mammalian subject such as a human body.

Fig. 2 is a schematic diagram illustrating an embodiment of a fluid balance monitoring system for determining and monitoring the fluid balance of a mammalian subject according to the invention.

Fig. 3 is a schematic diagram illustrating an embodiment of a method according to the invention.

**Description of Embodiments**

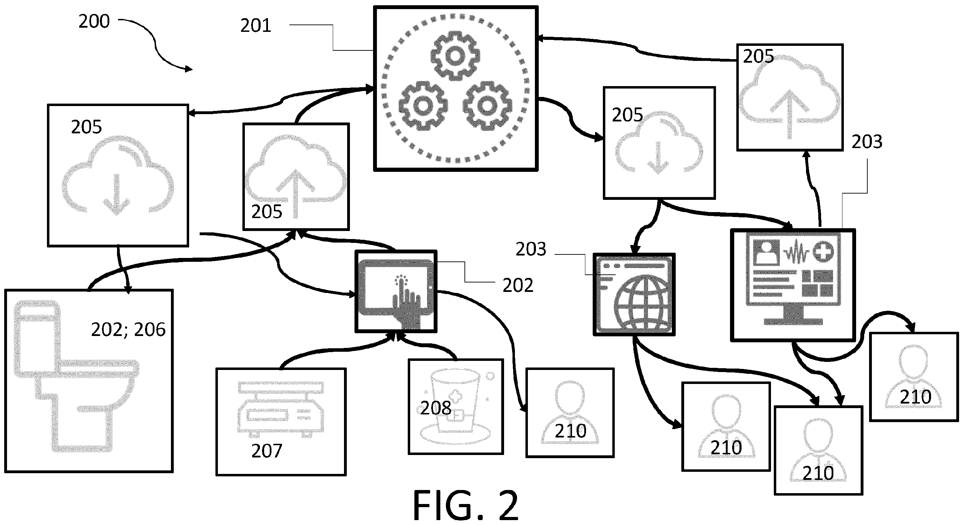

Referring to Fig. 2, a first embodiment of a fluid balance monitoring system 200 for determining and monitoring the fluid balance of a mammalian subject 100 (Fig. 1) according to the invention is shown.

The fluid balance monitoring system 200 according to the invention generally comprises a data processing unit 201 configured to manage and process measurement data, one or more input units 202 configured to receive measurement data and transmit the received measurement data to the data processing unit 201, and one or more display units 203 configured to receive output data indicative of the monitored fluid balance of the subject 100 and to display the output data indicative of the monitored fluid balance of the subject 100.

The one or more input units 202 are configured to receive measurement data and transmit the received measurement data to the data processing unit 201. The one or more input units 202 are in data transferring connection with the data processing unit 201. The one or more input units 202 may comprise a data processing unit. The one or more input units 202 may comprise a display. Generally, the data processing unit 201, the one or more input units 202 and the one or more display units 203 are mutually, particularly physically, separate units.

Generally, subjects 100, particularly humans, tend to move around and may thus experience inflow 101 and outflow 102 of liquids at different locations. For instance, subjects 100 generally defecate at different physical locations than where they eat. Thus, for easy registration of inflow 101 and outflow 102 of liquid, it is needed to enable recording of measurement data at different locations. The one or more input units 202 are therefore configured to enable collection of data at several different locations. Suitable input units 202 thus include a mobile telephone, a tablet computer and a laptop computer as well as other similar portable devices. The one or more input units 202 may receive measurement data by means of a user interface. Alternatively, or additionally, the one or more input units 202 may be configured to receive measurement data from sensors or other relevant devices, such as the devices 206, 207 and 208 shown in Fig. 2.

For instance, a toilet system 206 with sensors or an electronic scale 207 may be suitable for providing data indicative of fluid outflow 102. An electronic scale 207 may also provide data indicative of the weight and change in weight of the subject 100. Data indicative of fluid inflow 101 may for instance be measured by using, e.g., plates, glasses or cups 208 with built-in sensors of a suitable type, such as volume or weight sensors. Devices such as toilet systems 206, scales 207 and cups 208 as described above may also function as an input unit 202 in itself, for instance if being enabled for Internet of Things (IoT) or if comprising a data processing device or a data transmitter. Furthermore, it is feasible to preprogram software of the one or more input units 202 to enable display on a screen of the input unit 202, e.g. a touchscreen, of preset types and volumes, such that it is only time that is a novel variable.

It is noted in this connection that suitable toilet systems 206 with sensors are known in the art and include a toilet system as that described in the applicant's Danish patent application no DK PA 2020 70063, and/or as that described in US 2018/368818 A1.

Generally, the measurement data may comprise any data or information relevant for monitoring the fluid balance of a subject 100. The measurement data may comprise information regarding quantity, type and time for each event and individual subject 100. The information regarding quantity, type and time for each event may be saved together with or encrypted by an individual identification key to allow further summation for a specific individual.

An event may in this connection be any relevant event leading to an inflow 101 or an outflow 102 of fluid from a subject 100. Non-limiting examples include excretion, such as defecation and urination, output from drain intake of food or drink, exercise, perspiration, respiration, sputum secretion, mucosal secretion or gastric evacuation. The measurement data may also comprise data such as weight data regarding the subject 100.

The input unit 202 may further be configured to upload the measurement data to a cloud-based storage 205 or directly to a storage of the data processing unit 201. The input unit 202 may further be configured to provide the measurement data with an identification key for identifying the relevant subject 100 related to the measurements. The input unit 202 may still further be configured to receive data, for instance a request, from the data processing unit 201 via a cloud-based storage 205 or directly from the data processing unit 201. The request may be a request for providing new or further measurement data. The request may furthermore specify the measurement data required.

The data processing unit 201 is a central data processing unit in the sense that the fluid balance monitoring system 200 is configured such that almost all or all data communication between the one or more input units 202 and the one or more display units 203 is lead through the data processing unit 201. The data processing unit 201, the one or more input units 202 and the one or more display units 203 are thus mutually separate units. The fluid balance monitoring system 200 according to the invention is thus centrally managed.

The data processing unit 201 is configured to communicate with the one or more input units 202 and the one or more display units 203. The data processing unit 201 is configured to receive measurement data from the one or more input units 202 and to process the received measurement data to obtain output data indicative of the of the monitored fluid balance of the subject 100. The obtained output data indicative of the of the monitored fluid balance of the subject 100 may be a summarization of the data, e.g., in one or more tables or one or more graphs. The obtained output data may also be in any other format suitable for being displayed by the one or more display devices 203. The data processing unit 201 is further configured to transmit the obtained output data indicative of the of the monitored fluid balance of the subject 100 to the one or more display units 203. The data processing unit 201 is thus connected in a data transferring relationship with the one or more input units 202 and the one or more display units 203, where the connection may be wireless or wired, such as via a local area network (LAN) a wide area network (WAN) or the Internet.

The data processing unit 201 may still further be configured to transmit data, for instance a request, to the input unit 202 via a cloud-based storage 205 or directly to the input unit 202. The request may be a request for providing new or further measurement data. The request may furthermore specify the measurement data required. The request may be formed based on data, such as a request, received from the one or more display units 203. Alternatively, or additionally, the request may be sent provided no measurement data has been received at the data processing unit 201 for a predetermined amount of time.

The data transfer or transmission between the data processing unit 201 and the one or more input units 202 and one or more display units 203, respectively, may be direct or may be via intermediate storage, e.g. in a cloud-based storage 205. The data processing unit 201 may also comprise an internal storage. Data stored in a cloud-based storage 205 or in the internal storage of the data processing unit 201 may be associated with, e.g. stored together with or encrypted by, an individual identification key. The identification key may provide or comprise a suitable identification of a subject 100. The identification of the subject 100 may be anonymous such as to conform with relevant regulations, particularly data protection regulations such as the GDPR regulations in force within the European Union.

The one or more display units 203 are configured to receive the obtained output data indicative of the of the monitored fluid balance of the subject 100 from the data processing unit 201. The one or more display units 203 are in data transferring connection with the data processing unit 201. The one or more display units 203 are configured to display the obtained output data indicative of the of the monitored fluid balance of the subject 100. The one or more display units 203 thus comprise a display. More particularly, the one or more display units 203 are configured to display the obtained output data indicative of the of the monitored fluid balance of the subject 100 in a format suitable for easy and straight forward interpretation by a viewer 210. Such a format may for example include graph(s) or table(s). Typically, the viewer 210 is a health professional, such as a nurse or a doctor. As health professional are frequently on the move during their daily routines, suitable display units 203 include a mobile telephone, a tablet computer and a laptop computer as well as other similar portable devices. The one or more display units 203 may comprise a data processing unit, for instance to enable a final processing of the output data to allow displaying it. The one or more display units 203 may therefore in principle be any display unit comprising a processing unit connected to the data processing unit 201.

The one or more display units 203 may still further be configured to transmit data, for instance a request, to the data processing unit 201 via a cloud-based storage 205 or directly to the data processing unit 201. The request may be a request for providing new or further measurement data. The request may furthermore specify the measurement data required.

Since the measurement data are delivered to a central data processing unit 201 and processed to output data therein, processed measurement data originating from any input unit 202 may be displayed on any given display unit 203. The output data obtained by the data processing unit 201 and indicative of the of the monitored fluid balance of the subject 100 may thus be viewed on the display unit 203. The obtained output data may comprise any desired information. Examples are summarized data, like an overview of the fluid balance of the subject 100, total inflow 101 and outflow 102, and the contribution of each measuring unit or measurement to the total fluid balance. Likewise, the fluid balance of the subject 100 for a given period can be displayed on the display unit 203. The display unit 203 may further be configured to update the displayed data continuously or with a determined time interval.

In the above description, the display unit 203, the input unit 202 and the data processing unit 201 are envisaged to be different physical units. It is noted, however, that the display unit 203 and the input unit 202 may also be the same physical unit. It is even possible that the display unit 203, the input unit 202 and the data processing unit 201 may be one and the same physical unit. In both cases the units may still be set up to ensure that all data communication goes through the data processing unit 201. Irrespective of the embodiment, the display unit 203 and the input unit 201 are synchronized.

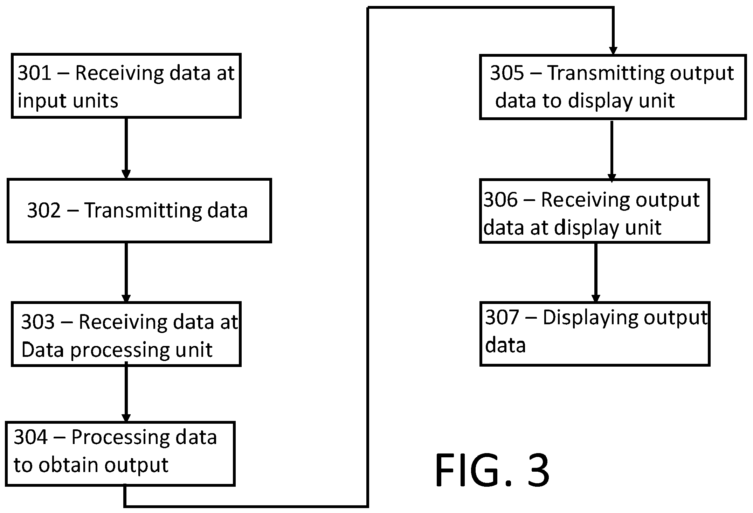

Turning now to Fig. 3, a method for determining and monitoring the fluid balance of a mammalian subject 100 according to the invention will be described. The method comprises the following steps.

First, a fluid balance monitoring system 200 according to the invention is provided. The fluid balance monitoring system 200 comprises at least one data processing unit 201 with at least one data processing device, at least one input unit 202 and at least one display unit 203 with at least one data processing device.

In step 301, the at least one input unit 202 is used to receive, from any one or more of a user interface and at least one sensor device 206, 207, 208, measurement data comprising information relevant for determining and monitoring the fluid balance of a subject 100. The measurement data comprises information regarding a quantity, a type and a time of occurrence for at least one event relevant for determining and monitoring the fluid balance of a subject 100, where the at least one event is any one relevant event leading to an outflow of fluid from the subject or an inflow of fluid to the subject 100.

In step 302, the at least one input unit 202 is used to transmit the received measurement data to the at least one data processing unit 201.

In step 303, the at least one data processing unit 201 receives the measurement data from the at least one input unit 202.

In step 304, the at least one data processing unit 201 is used to process the received measurement data to achieve output data indicative of the fluid balance of a subject 100.

In step 305, the at least one data processing unit 201 transmits the output data indicative of the fluid balance of a subject 100 to the at least one display unit 203.

In step 306, the at least one display unit 203 receives the output data indicative of the fluid balance of a subject 100.

Finally, in step 307, the at least one display unit 203 displays the output data indicative of the fluid balance of a subject 100 and further indicative of a type and a time of occurrence of the at least one event.

In a further, optional, step data, such as measurement data or the output data indicative of the fluid balance of a subject 100, may be uploaded to or saved in the subject's case sheet.

The person skilled in the art realizes that the present invention by no means is limited to the preferred embodiments described above. On the contrary, many modifications and variations are possible within the scope of the appended claims.

List of reference numerals

| 100 | Subject |
| 101 | Liquid input |
| 102 | Liquid output |

| 200 | Fluid balance monitoring system |
| 201 | Data processing unit |
| 202 | Input unit |
| 203 | Display unit |
| 204 | Display unit |
| 205 | Cloud-based storage |
| 206 | Toilet system with sensors |
| 207 | Electronic scale |
| 208 | Cup with sensors |
| 210 | Healthcare professional/Viewer |

| 301-307 | Method steps |

**Claims**

1. A fluid balance monitoring system (200) adapted for determining and monitoring the fluid balance of a mammalian subject (100), the fluid balance monitoring system comprising at least one data processing unit (201), at least one input unit (202) and at least one display unit (203),

   wherein the at least one input unit (202) is configured to:

   - receive, from any one or more of a user interface and at least one sensor device (206, 207, 208), measurement data comprising information relevant for determining and monitoring the fluid balance of a mammalian subject, and
   - transmit the received measurement to the at least one data processing unit,

wherein the at least one data processing unit (201) comprises a data processing device, and the at least one data processing unit is configured to:

- receive the measurement data from the at least one input unit,
- process the received measurement data to achieve output data indicative of the fluid balance of the mammalian subject, and
- transmit the output data indicative of the fluid balance of a subject to the at least one display unit,

wherein the at least one display unit (203) comprises a data processing device and the at least one display unit is configured to:

- receive the output data indicative of the fluid balance of the mammalian subject, and
- display the output data indicative of the fluid balance of the mammalian subject,

wherein the at least one data processing unit (201), the at least one input unit (202) and the at least one display unit (203) are mutually, particularly physically, separate units,
wherein the measurement data further comprises information regarding a quantity, a type and a time of occurrence for at least one event relevant for determining and monitoring the fluid balance of the mammalian subject (100), where the at least one event is any one relevant event leading to an outflow of fluid from the mammalian subject or an inflow of fluid to the mammalian subject, and
wherein the at least one data processing unit (201) furthermore is configured to process the received measurement data to achieve output data which is further indicative of a type and a time of occurrence of the at least one event.

2. A fluid balance monitoring system according to claim 1, wherein the at least one data processing unit (201) is in data transferring connection with the at least one input unit (202) and the at least one display unit (203) in such a way that all data communication between the at least one input unit and the at least one display unit is lead through the at least one data processing unit.

3. A fluid balance monitoring system according to any one of the above claims, wherein the measurement data comprises data related to at least one event relevant for determining and monitoring the fluid balance of the mammalian subject, where the at least one event includes any one or more of: excretion, defecation, urination, intake of food or drink, intravenous treatment, subcutaneous treatment, exercise, output from drain, output from ulcers, perspiration, respiration, sputum secretion, mucosal secretion, and gastric evacuation.

4. A fluid balance monitoring system according to any one of the above claims, wherein at least one of the measurement data and the output data is saved in a cloud-based storage (205) or a storage provided in the data processing unit, preferably together with or encrypted by an individual identification key.

5. A fluid balance monitoring system according to any one of the above claims, wherein the output data comprises at least data indicative of an inflow of liquid to the mammalian subject, an outflow of liquid from the mammalian subject and a change in the fluid balance of the mammalian subject.

6. A fluid balance monitoring system according to claim 5, wherein the display unit (203) is configured to display the output data in a manner showing the data indicative of an inflow of liquid to the mammalian subject, an outflow of liquid from the mammalian subject and a change in the fluid balance of the mammalian subject separately from one another.

7. A fluid balance monitoring system according to any one of the above claims, wherein the data processing unit (201) is configured to generate output data and send the output data to the at least one display unit in real time or with predetermined time intervals, and/or wherein the display unit (203) is configured to display the output data in real time or with predetermined time intervals.

8. A method for determining and monitoring the fluid balance of a mammalian subject (100), the method comprising the steps of:

a) providing a fluid balance monitoring system (200) according to any one of the above claims and comprising at least one data processing unit (201) with at least one data processing device, at least one input unit (202)

and at least one display unit (203) with at least one data processing device,

b) receiving (301), with the at least one input unit and from any one or more of a user interface and at least one sensor device, measurement data comprising information relevant for determining and monitoring the fluid balance of a mammalian subject, and

c) transmitting (302), with the at least one input unit, the received the measurement data to the at least one data processing unit,

d) receiving (303), with the at least one data processing unit, the measurement data from the at least one input unit,

e) processing (304), with the at least one data processing unit, the received measurement data to achieve output data indicative of the fluid balance of the mammalian subject,

f) transmitting (305), with the at least one data processing unit, the output data indicative of the fluid balance of the mammalian subject to the at least one display unit,

g) receiving (306), with the at least one display unit, the output data indicative of the fluid balance of the mammalian subject, and

h) displaying (307), with the at least one display unit, the output data indicative of the fluid balance of the mammalian subject,

wherein the at least one data processing unit (201), the at least one input unit (202) and the at least one display unit (203) are mutually, particularly physically, separate units,

wherein the measurement data further comprises information regarding a quantity, a type and a time of occurrence for at least one event relevant for determining and monitoring the fluid balance of the mammalian subject (100), where the at least one event is any one relevant event leading to an outflow of fluid from the mammalian subject or an inflow of fluid to the mammalian subject, and

comprising the further step of processing, with the at least one data processing unit (201), the received measurement data to achieve output data which is further indicative of a type and a time of occurrence of the at least one event.

9. A method according to claim 8, wherein:

the at least one data processing unit (201) is in data transferring connection with the at least one input unit (202) and the at least one display unit (203) in such a way that all data communication between the at least one input unit and the at least one display unit is lead through the at least one data processing unit, or

wherein the first measurement data and second measurement data comprises data related to at least one event relevant for determining and monitoring the fluid balance of the mammalian subject, where the at least one event includes any one or more of: excretion, defecation, urination, intake of food or drink, exercise, perspiration and respiration, or

wherein the output data comprises at least data indicative of an inflow of liquid to the mammalian subject, an outflow of liquid from the mammalian subject and a change in the fluid balance of the mammalian subject.

10. A method according to claim 8 or 9, and comprising one or more of the further steps of:

saving at least one of the measurement data and the output data in a cloud-based storage (205) or a storage provided in the data processing unit, preferably together with or encrypted by an individual identification key, generating, with the data processing unit (201), output data in real time and sending, with the data processing unit, the output data to the at least one display unit in real time or with predetermined time intervals, and displaying, with the display unit (203), the output data in real time or with predetermined time intervals.

**100**

**101**

**102**

# FIG. 1

FIG. 2

305 – Transmitting output data to display unit

306 – Receiving output data at display unit

307 – Displaying output data

301 – Receiving data at input units

302 – Transmitting data

303 – Receiving data at Data processing unit

304 – Processing data to obtain output

FIG. 3

## REFERENCES CITED IN THE DESCRIPTION

**Patent documents cited in the description**

- DK PA202070063

- US 2018368818 A1